# EUROPEAN PATENT APPLICATION

(11) **EP 3 235 609 A1**
(43) Date of publication of application: **25.10.2017**
(21) Application number: 15870017.9
(22) Date of filing: 16.12.2015
(51) Int. Cl.: B25J 18/02

(54) **ROBOT ARM MECHANISM**

(30) Priority: 20.12.2014 JP 2014258147
(71) Applicant: Life Robotics Inc., Koto-ku Tokyo 135-0047 (JP)
(72) Inventor: YOON Woo-Keun, Tokyo 135-0047 (JP)
(74) Representative: Herrero & Asociados, S.L.
(86) International application number: PCT/JP2015/085236
(87) International publication number: WO 2016/098815

(57) **Abstract**

A rigidity of a robot arm mechanism including a linear extension and retraction joint is enhanced. In a robot arm mechanism including the linear extension and retraction joint, the linear extension and retraction joint includes an arm section (2) and an ejection section (30), the arm section (2) includes a first connection piece string (21) including a plurality of first connection pieces (23) and a second connection piece string (20) including a plurality of second connection pieces (22), and the second connection piece string (20) is joined to the first connection piece string (21) to thereby constitute a columnar body having a certain rigidity. The ejection section (30) includes lower rollers (311) and upper rollers (312) for joining the first and second connection piece strings (21, 20) and supporting the columnar body. The lower rollers (311) and the upper rollers (312) are disposed with the columnar body sandwiched between the lower rollers (311) and the upper rollers (312).

## Description

### FIELD

Embodiments described herein relate generally to a robot arm mechanism.

### BACKGROUND

Conventionally, an articulated robot arm mechanism is used in various fields such as an industrial robot. For example, the articulated robot arm mechanism like this is provided with a linear extension and retraction joint in combination with other joints. An arm section constituting the linear extension and retraction joint includes, for example, connection piece strings in each of which a plurality of pieces having the same shapes are connected in a string shape. The connection piece strings are joined, and thereby a columnar body having a certain rigidity is formed. When the linear extension and retraction joint is driven, the arm section that is formed as a columnar body is sent out. When a force that holds the columnar body is small, the rigidity of the robot arm mechanism is reduced.

### BRIEF DESCRIPTION OF THE INVENTION

A purpose of the present invention is to enhance rigidity of a robot arm mechanism having a linear extension and retraction joint.

The robot arm mechanism according to the present embodiment includes a linear extension and retraction joint, the linear extension and retraction joint includes an arm section and an ejection section for supporting the arm section, the arm section includes a first connection piece string and a second connection piece string, the first connection piece string includes a plurality of first connection pieces each having a U-shaped cross section or a hollow square cross section, the second connection piece string includes a plurality of second connection pieces each having a substantially flat plate shape, the second connection piece string is joined to the first connection piece string to thereby constitute a columnar body having a certain rigidity, the ejection section includes first rollers and second rollers for joining the first and second connection piece strings and supporting the columnar body by joining of the first and second connection piece strings, and the first rollers and the second rollers are disposed with the columnar body sandwiched between the first rollers and the second rollers.

### BRIEF DESCRIPTION OF THE VIEWS OF THE DRAWINGS

FIG. 1 is an external perspective view of a robot arm mechanism according to the present embodiment;
FIG. 2 is a perspective view illustrating an internal structure of the robot arm mechanism in FIG. 1;
FIG. 3 is a view illustrating the internal structure of the robot arm mechanism in FIG. 1, which is a cross section view;
FIGS. 4A and 4B are views illustrating an internal structure of an ejection section in FIG. 3;
FIGS. 5A to 5C are diagrams of the ejection section in FIG. 3, which are a top diagram, a side diagram, and a bottom diagram;
FIGS. 6A to 6D are supplementary explanatory diagrams for explaining a joining function of the ejection section of the robot arm mechanism according to the present embodiment;
FIGS. 7A and 7B are cross section views of an ejection section according to a modified example, which is cut along a section orthogonal to an arm center axis;
FIGS. 8A to 8C are diagrams of the ejection section in FIGS. 7A and 7B, which are a top diagram, a side diagram, and a bottom diagram;
FIGS. 9A and 9B are supplementary explanatory diagrams for explaining a disposition condition of a plurality of upper rollers and a plurality of lower rollers that constitute the ejection section according to the present embodiment;
FIGS. 10A to 10C are views illustrating modified examples of the ejection section in FIGS. 9A and 9B; and
FIGS. 11A and 11B are views illustrating examples of a structure of an ejection section 30 in the case of using a single upper roller and a single lower roller.

### DETAILED DESCRIPTION

Hereinafter, a robot arm mechanism according to the present embodiment is described with reference to the accompanying drawings. In the following description, the same reference numerals denote components having substantially identical functions and structures, and the repeated description thereof is made only when necessary.

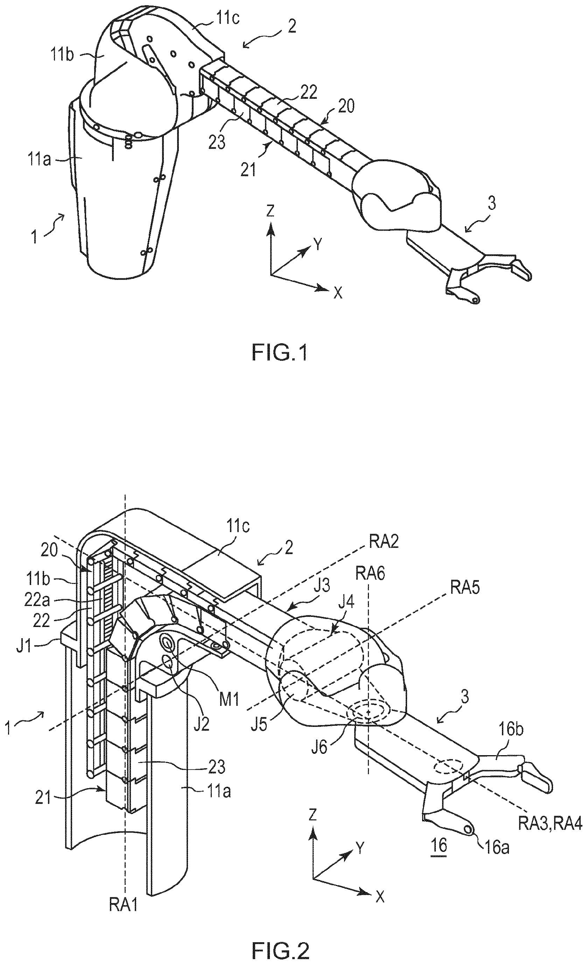

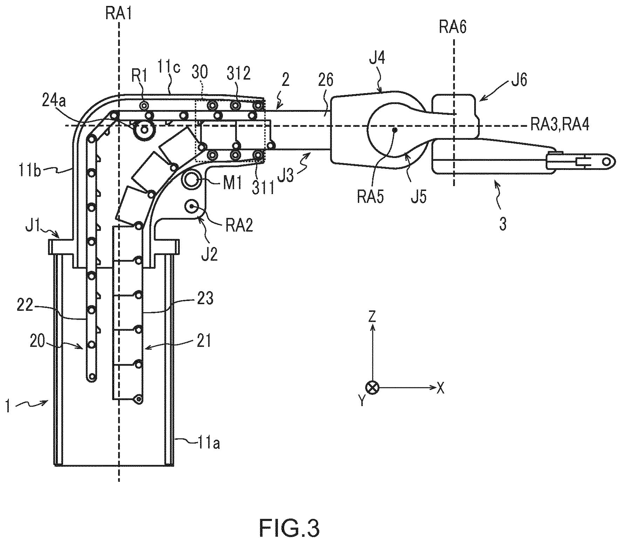

FIG. 1 is an external perspective view of the robot arm mechanism according to the present embodiment. FIG. 2 and FIG. 3 are views illustrating an internal structure of the robot arm mechanism in FIG. 1. The robot arm mechanism includes a substantially cylindrical base 1 and an arm section 2 connected to the base 1. An end effector 3 is attached to a tip of the arm section 2. In FIG. 1, a hand section capable of holding an object is illustrated as the end effector 3. The end effector 3 is not limited to the hand section, but may be another tool, a camera, or a display. At the tip of the arm section 2, an adapter which can be replaced with any type of the end effector 3 may be provided.

The arm section 2 includes a plurality (herein, six) of joints J1, J2, J3, J4, J5 and J6. The plurality of the joints J1, J2, J3, J4, J5 and J6 are arranged in order from the base 1. Generally, a first axis RA1, a second axis RA2, and a third axis RA3 are called root three axes, and a fourth axis RA4, a fifth axis RA5, and a sixth axis RA6 are called wrist three axes for changing the posture of the hand section 3. At least one of the joints J1, J2 and J3 constituting the root three axes is a linear motion joint. Herein, the third joint J3 is formed as a linear motion joint, in particular, a joint with a relatively long extension distance. The first joint J1 is a torsion joint that rotates on the first axis of rotation RA1 which is held, for example, perpendicularly to a base surface. The second joint J2 is a bending joint that rotates on the second axis of rotation RA2 perpendicular to the first axis of rotation RA1. The third joint J3 linearly extends or retracts along the third axis (axis of movement) RA3 perpendicular to the second axis of rotation RA2. The fourth joint J4 is a torsion joint that rotates on the fourth axis of rotation RA4 which matches the third axis of movement RA3. The fifth joint J5 is a bending joint that rotates on the fifth axis of rotation RA5 orthogonal to the fourth axis of rotation RA4. The sixth joint J6 is a bending joint that rotates on the sixth axis of rotation RA6 orthogonal to the fourth axis of rotation RA4 and perpendicular to the fifth axis of rotation RA5.

The arm section 2 turns together with the hand section 3 in accordance with torsional rotation of the first joint J1. The arm section 2 rotates upward and downward on the second axis of rotation RA2 of the second joint J2 together with the hand section 3 in accordance with bending rotation of the second joint J2. An arm support body (a first support body) 11a forming the base 1 has a cylindrical hollow structure formed around the axis of rotation RA1 of the first joint J1. The first joint J1 is mounted on a fixed base (not shown). When the first joint J1 rotates, the first support body 11a axially rotates in accordance with the turn of the arm section 2. The first support body 11a may be fixed on a ground plane. In this case, the arm section 2 turns independently of the first support body 11a. A second support body 11b is connected to an upper part of the first support body 11a.

The second support body 11b has a hollow structure continuous to the first support body 11a. One end of the second support body 11b is attached to a rotating section of the first joint J1. The other end of the second support body 11b is opened, and a third support body 11c is set rotatably on the axis of rotation RA2 of the second joint J2. The third support body 11c has a scaly hollow structure communicating with the first support body 11a and the second support body 11b. In accordance with the bending rotation of the second joint J2, a rear part of the third support body 11c is accommodated in or sent out from the second support body 11b. The rear part of the third joint J3, which constitutes the linear motion joint of the arm section 2, is housed inside the continuous hollow structure of the first support body 11a and the second support body 11b by retraction thereof.

The first joint J1 includes an annular fixed section and a rotating section, and is fixed to a base at the fixed section. The first support body 11a and the second support body 11b are attached to the rotating section. When the first joint J1 rotates, the first support body 11a, the second support body 11b, and the third support body 11c turn around the first axis of rotation RA1 together with the arm section 2 and the hand section 3.

The third support body 11c is set rotatably, at the lower part of its rear end, on the axis of rotation RA2 with respect to a lower side of an open end of the second support body 11b. In this way, the second joint J2 serving as a bending joint that rotates on the axis of rotation RA2 is formed. When the second joint J2 rotates, the arm section 2 rotates vertically, i.e., rotates upward and downward, on the axis of rotation RA2 of the second joint J2 together with the hand section 3. The axis of rotation RA2 of the second joint J2 is perpendicular to the first axis of rotation RA1 of the first joint J1 serving as a torsion joint.

As described above, the third joint J3 serving as a joint constitutes a main constituent of the arm section 2. The hand section 3 described above is provided at the tip of the arm section 2. The hand section 3 is equipped at the tip of the arm section 2 as illustrated in FIG. 1. The hand section 3 is moved to a given position by the first joint J1, the second joint J2, and the third joint J3, and placed in a given posture by the fourth joint J4, the fifth joint J5, and the sixth joint J6. The hand section 3 includes two fingers 16a and 16b configured to be opened and closed. The fourth joint J4 is a torsion joint having the axis of rotation RA4 which typically matches a center axis of the arm section 2 along the extension and retraction direction of the arm section 2, that is, the axis of movement RA3 of the third joint J3. When the fourth joint J4 rotates, the hand section 3 rotates on the axis of rotation RA4 from the fourth joint J4 to the tip thereof.

The fifth joint J5 is a bending joint having the axis of rotation RA5 orthogonal to the axis of movement RA4 of the fourth joint J4. When the fifth joint J5 rotates, the hand section 3 pivots up and down from the fifth joint J5 to its tip together with a hand 16. The sixth joint J6 is a bending joint having the axis of rotation RA6 orthogonal to the axis of rotation RA4 of the fourth joint J4 and perpendicular to the axis of rotation RA5 of the fifth joint J5. When the sixth joint J6 rotates, the hand 16 turns left and right.

Rotation, bending, and extension and retraction of the first to sixth joints J1 to J6 enable positioning a two-fingered hand 16 of the hand section 3 at a given position and posture. In particular, the linear extension and retraction distance of the third joint J3 enables the hand section 3 to act on an object in a wide range from a position close to the base 1 to a position far from the base 1.

The third joint J3 is characterized by the linear extension and retraction distance realized by a linear extension and retraction arm mechanism constituting the third joint J3. The linear extension and retraction distance is achieved by the structure shown in FIG. 2 and FIG. 3. The linear extension and retraction arm mechanism includes a first connection piece string 21 and a second connection piece string 20. In an alignment pose where the arm section 2 is horizontal, the first connection piece string 21 is located below the second connection piece string 20, and the second connection piece string 20 is located above the first connection piece string 21. A back surface side of a first connection piece 23 faces a back surface side of a second connection piece 22.

The first connection piece string 21 includes a plurality of first connection pieces 23 having the same U-shaped cross section and connected to form a string by pins at their front surface parts. The first connection piece string 21 is bendable in its back surface direction but conversely not bendable in its front surface direction due to the shape of the cross section of the first connection piece 23 and connection positions by the pins. Therefore, the shape of the cross section of the first connection piece 23 may be a hollow square shape, etc. as well as the U shape.

The second connection piece string 20 includes a plurality of second connection pieces 22 having a substantially flat plate shape with a width substantially equivalent to that of the first connection piece 23, and connected to form a string by pins in a bendable state in both a back surface direction and a front surface direction. The first connection piece string 21 is joined to the second connection piece string 20 at the tip of the first connection piece string 21 by a joining piece 26. The joining piece 26 has an integrated shape of the first connection piece 23 and the second connection piece 22. As shown in FIG. 2, a linear gear 22a is formed on the back surface of each of the second connection pieces 22. The linear gears 22a are connected to form a continuous linear gear (rack) when the second connection piece 22 has a linear shape.

As shown in FIG. 3, the second connection piece 22 is sandwiched between a roller R1 and a drive gear 24a in the third support body 11c. Thereby, the linear gear 22a is engaged with the drive gear 24a. When the arm is extended, a motor M1 is driven, and the drive gear 24a rotates forward, so that the second connection piece string 20 becomes a columnar body together with the first connection piece string 21 and is sent out forward from the ejection section 30. At that time, the back surface of the first connection piece string 21 and the back surface of the second connection piece string 20 are joined to each other. When the arm is retracted, the motor M1 is driven, and the drive gear 24a rotates backward, so that the joined state of the strings 20 and 21 is released behind the ejection section 30, and the first connection piece string 21 and the second connection piece string 20 are separated from each other. The second connection piece string 20 and first connection piece string 21 separated from each other restore their bendable state, are bent in a direction along the first axis of rotation RA1, and are housed inside the first support body 11a.

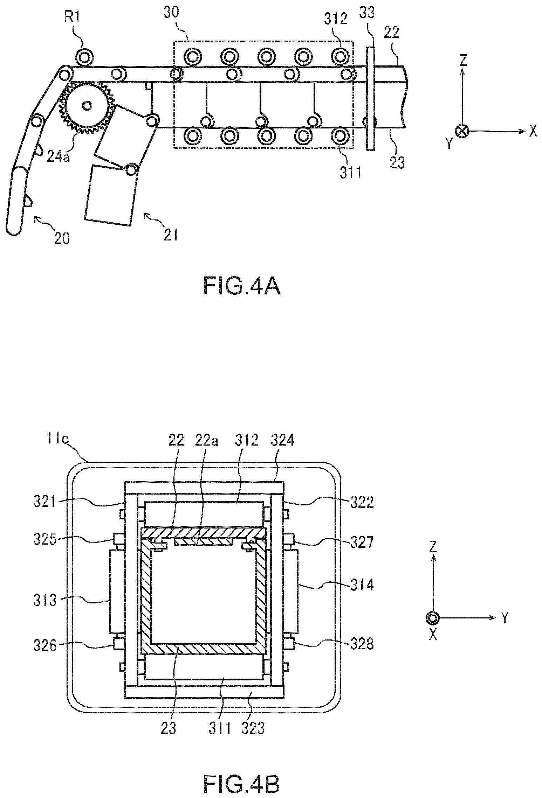

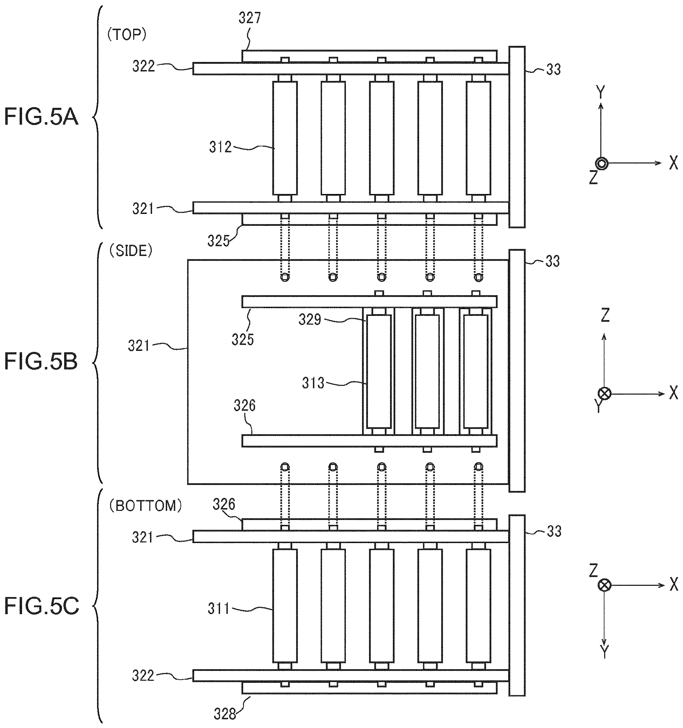

Hereinafter, a structure of the ejection section 30 will be described with reference to FIGS. 4A and 4B and 5A to 5C. FIGS. 4A and 4B are views illustrating an internal structure of the ejection section in FIG. 3. FIG. 4A is a view of the ejection section 30, which is a cross section view. FIG. 4B is a cross section view of the ejection section 30 which is cut along a section orthogonal to an ejection center axis. FIGS. 5A to 5C are diagrams of the ejection section 30, which are a top diagram, a side diagram, and a bottom diagram. FIG. 5A is a diagram of the ejection section 30 seen from above. FIG. 5B is a diagram of the ejection section 30 seen from a side. FIG. 5C is a diagram of the ejection section 30 seen from below.

The ejection section 30 includes a structure for joining the first connection piece string 21 and the second connection piece string 20 and supporting the arm section 2. Hereinafter, a typical structure of the ejection section 30 is described. The ejection section 30 is disposed in a vicinity of a rear part of an ejection port 33. The ejection section 30 has an external appearance in a substantially rectangular cylindrical shape. Hereinafter, a center axis of the ejection section 30 in the substantially rectangular cylindrical shape is referred to as an ejection center axis. The ejection center axis corresponds to an arm center axis. The arm center axis is a center axis of the columnar body that is supported by the ejection section 30.

The ejection section 30 includes a pair of side wall plates 321 and 322, a lower wall plate 323, and an upper wall plate 324. Planes of the side wall plates 321 and 322 are rectangular flat plates. The pair of side wall plates 321 and 322 are respectively disposed by being separated by equal distances in a lateral direction (a +Y direction and a -Y direction in the drawings) from the ejection center axis. The pair of side wall plates 321 and 322 are separated by a longer distance than the width of the columnar body. A space between lower end portions of the pair of side wall plates 321 and 322 is fixed by the lower wall plate 323, and a space between upper end portions is fixed by the upper wall plate 324. The space between the pair of side wall plates 321 and 322 is kept constant by the lower wall plate 323 and the upper wall plate 324. The pair of side wall plates 321 and 322 are fixed to the first support body 11a or the second support body 11b.

The side wall plate 321 is provided with a plurality of side rollers 313, and the side wall plate 322 is provided with a plurality of side rollers 314. Hereinafter, the plurality of side rollers 313 that are provided on the side wall plate 321 are described.

A pair of bearing portions 325 and 326 are fixed to predetermined positions on an outer side surface of the side wall plate 321. The bearing portion 325 is a slim rectangular column, and has a center axis parallel with the ejection center axis. The bearing portion 326 has the same shape as that of the bearing portion 325. The positions to which the pair of bearing portions 325 and 326 are fixed respectively correspond to positions that are separated by equal distances in a vertical direction (a +Z direction and -Z direction in the drawings) from the ejection center axis.

The plurality of side rollers 313 are rotatably fixed to between the bearing portions 325 and 326. The plurality of side rollers 313 are arranged parallel with one another along the ejection center axis. The plurality of side rollers 313 have axes of rotation in the vertical direction (the Z direction in the drawings). The side roller 313 has a longer radius than a distance to an inner side surface of the side wall plate 321 from the axis of rotation of the side roller 313 that is fixed to the bearing portions 325 and 326. In a predetermined position of the side wall plate 321, a projection hole 329 for allowing the side roller 313 to project from the inner side surface of the side wall plate 321 is formed. A position where the projection hole 329 is formed in the side wall plate 321 corresponds to a position of the side roller 313 that is fixed to between the bearing portions 325 and 326. Since the projection hole 329 is formed in the side wall plate 321, the side roller 313 fixed to the bearing portions 325 and 326 projects from the inner side surface of the side wall plate 321. A support surface that supports the columnar body is defined by projection ends of the plurality of side rollers 313 that are projected from the inner side surface of the side wall plate 321.

In the same way as the side wall plate 321, a pair of bearing portions 327 and 328 are fixed to the side wall plate 322. In the same way as the pair of bearing portions 325 and 326, a plurality of side rollers 314 are rotatably fixed to between the pair of bearing portions 327 and 328. The side roller 314 has the same shape as the side roller 313. The plurality of side rollers 314 project from an inner side surface of the side wall plate 322 in the same way as the plurality of side rollers 313. A support surface that supports the columnar body is defined by projection ends of the plurality of side rollers 314 that are projected from the inner side surface of the side wall plate 322.

A distance from the support surface defined by the plurality of side rollers 313 to the support surface defined by the plurality of side rollers 314 corresponds to a width of a cylindrical part of the ejection section 30. The support surface defined by the plurality of side rollers 313 and the support surface defined by the plurality of side rollers 314 are designed so that the width of the cylindrical part of the ejection section 30 is equal to or less than a width of the columnar body. Specifically, projection lengths of the side rollers 313 and 314 from the inner side surfaces of the side wall plates 321 and 322 are adjusted so that the distance from the support surface defined by the plurality of side rollers 313 to the support surface defined by the plurality of side rollers 314 becomes equal to or less than the width of the columnar body. When the distance from the support surface defined by the plurality of side rollers 313 to the support surface defined by the plurality of side rollers 314 is shorter than the width of the columnar body, the plurality of side rollers 313 and 314 support the columnar body in a state where a preload is applied to between the plurality of side rollers 313 and 314 and the columnar body.

A plurality of upper rollers 312 are rotatably fixed to between upper parts of the side wall plates 321 and 322. The plurality of upper rollers 312 are arranged parallel with one another along the ejection center axis. Accordingly, the plurality of upper rollers 312 have axes of rotation in the lateral direction (the Y direction in the drawings). A support surface that supports the columnar body is defined by lower portions of the plurality of upper rollers 312. A plurality of lower rollers 311 are rotatably fixed to between lower parts of the side wall plates 321 and 322. The plurality of lower rollers 311 are arranged parallel with one another along the ejection center axis. Accordingly, the plurality of lower rollers 311 have axes of rotation in the lateral direction (the Y direction in the drawings). A support surface that supports the columnar body is defined by upper portions of the plurality of lower rollers 311.

A distance from the support surface defined by the plurality of upper rollers 312 to the support surface defined by the plurality of lower rollers 311 corresponds to a thickness of the cylindrical part of the ejection section 30. The support surface defined by the plurality of upper rollers 312 and the support surface defined by the plurality of lower rollers 311 are designed so that the thickness of the cylindrical part of the ejection section 30 is equal to or less than a thickness of the columnar body. Specifically, fixed positions of the plurality of upper rollers 312 and the plurality of lower rollers 311 are determined so that the distance from the support surface defined by the plurality of upper rollers 312 to the support surface defined by the plurality of lower rollers 311 becomes equal to or less than the thickness of the columnar body. When the distance from the support surface defined by the plurality of upper rollers 312 to the support surface defined by the plurality of lower rollers 311 is shorter than the thickness of the columnar body, the plurality of upper rollers 312 and the plurality of lower rollers 311 support the columnar body in a state where a preload is applied to between the plurality of upper rollers 312 and the plurality of lower rollers 311, and the columnar body.

As described above, the ejection section 30 has a substantially rectangular cylindrical shape. The columnar body is supported by the cylindrical part of the ejection section 30. The width and the thickness of the cylindrical part of the ejection section 30 are designed so as to be smaller than the width and the thickness of the columnar body respectively. This brings about the state where a preload is applied to between the ejection section 30 and the columnar body. An appropriate preload is applied to between the ejection section 30 and the columnar body, and thereby a force with which the ejection section 30 supports the columnar body can be enhanced. Accordingly, rigidity of the ejection section 30 can be increased. Further, rattling at a time of retraction of the arm is reduced, and as a result of rattling being reduced, strange sound can be prevented. That is, a deformation amount of the ejection section 30 can be decreased with respect to an exerted external force accompanying movement of the arm section 2 and the end effector 3 and an external force corresponding to weights of the arm section 2 and the end effector 3. Thereby, precision with which the position of the arm section 2 is held by the ejection section 30 can be enhanced. In the present embodiment, a fixed position preload is adopted as a method for applying a preload. In the present embodiment, the fixed position preload refers to a method for applying a preload to between the columnar body and the ejection section 30 based on a positional relationship between the rollers and the columnar body. At this time, the space between the rollers is set to be slightly shorter than the width (the thickness) of the columnar body, and thereby a preload is applied to between the columnar body and the ejection section 30. However, the method for applying a preload to between the columnar body and the ejection section 30 is not limited to this method. A preload may be applied to between the columnar body and the ejection section 30 by a constant-pressure preload using a coil spring, a leaf spring, etc.. In the present embodiment, a preload can be applied to between the columnar body and the ejection section 30 by pushing the rollers to the columnar body by using these springs.

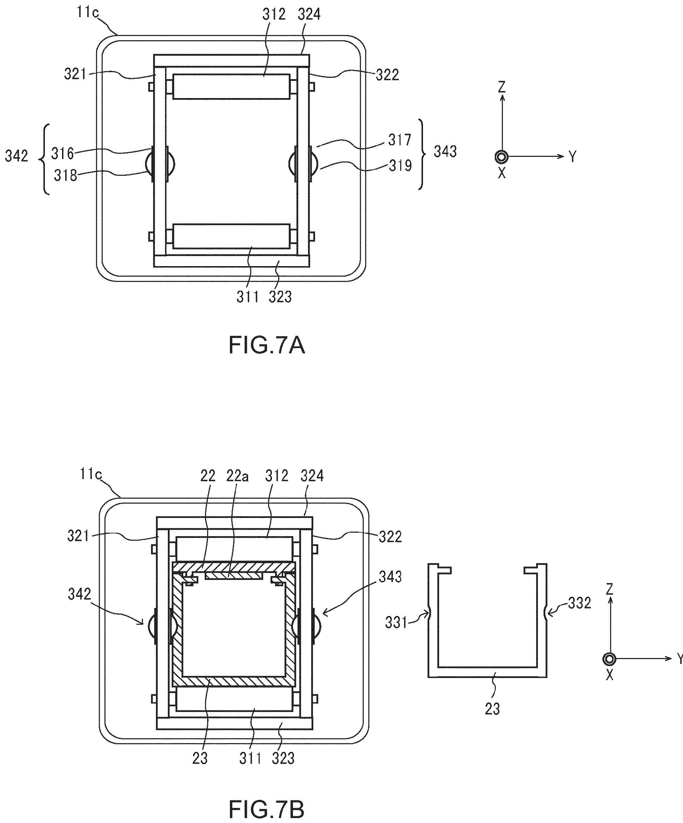

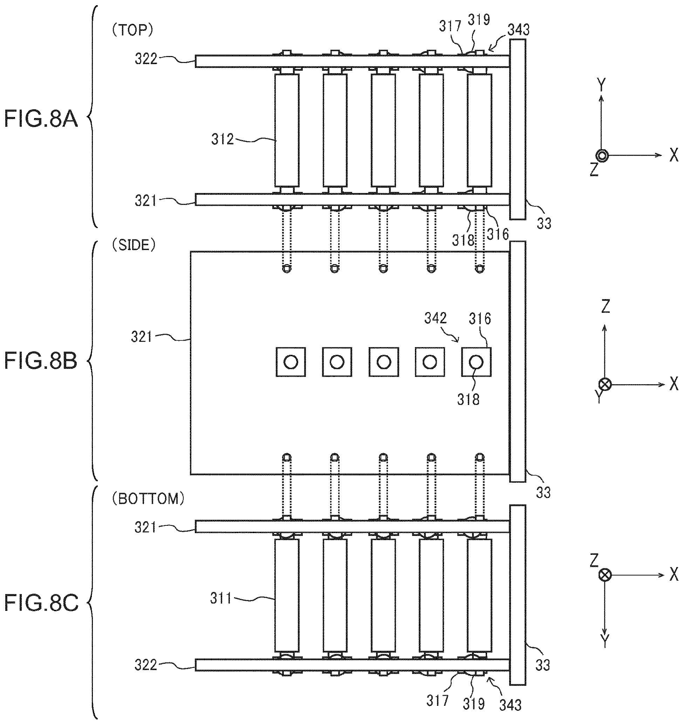

On the ejection section 30, an external force (the gravity) corresponding to the weights of the arm section 2 and the end effector 3 always work. Accordingly, an effect of a preload being applied to between the ejection section 30 and the columnar body is remarkable especially concerning the upper rollers 312 and the lower rollers 311 of the ejection section 30. On the other hand, a large external force does not work on the side rollers 313 and 314 as compared with the upper rollers 312 and the lower rollers 311. Accordingly, even when a preload is not applied to between the side rollers 313 and 314, and the columnar body, the rigidity of the ejection section 30 is not reduced significantly. That is, the width of the cylindrical part of the ejection section 30 may have any dimension as long as it is equivalent to the width of the columnar body. Further, in place of the side rollers 313 and 314 that are in line contact with the columnar body, ball bearings that are in point contact with the columnar body may be used. A structure of the ejection section 30 at the time of ball bearings being used in place of the side rollers 313 and 314 is described later with reference to FIGS. 7A and 7B, and FIGS. 8A to 8C.

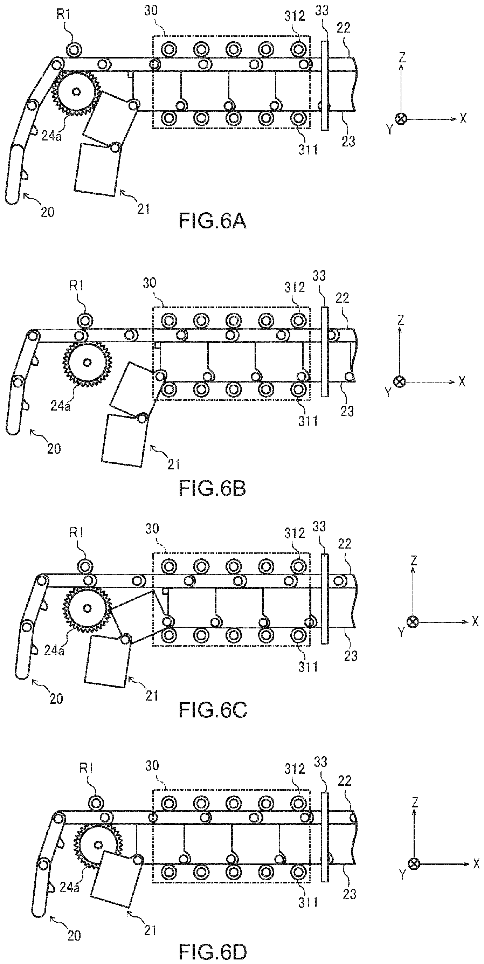

A joining function of the ejection section 30 is described with reference to FIGS. 6A to 6D.

The ejection section 30 has the joining function of joining the first connection piece string 21 and the second connection piece string 20. FIGS. 6A to 6D are supplementary explanatory diagrams for explaining the joining function of the ejection section 30 of the robot arm mechanism according to the present embodiment. FIGS. 6A to 6D are diagrams showing a state in which the first connection piece string 21 and the second connection piece string 20 are guided to the ejection section 30 and are joined to each other, when the arm section 2 is extended, at a lapse of each unit time. An elapsed time period is expressed in order from FIG. 6A to FIG. 6D.

When the drive gear 24a rotates forward, the second connection piece string 20 is guided to the ejection section 30 together with the first connection piece string 21. The first connection piece 23 guided to the ejection section 30 is aligned on a straight line parallel to the ejection center axis while rotating along the last lower roller 311. In the same way, the second connection piece 22 that is guided to the ejection section 30 is aligned on a straight line parallel to the ejection center axis along the axis of rotation of the last upper roller 312. As described above, the thickness of the cylindrical part of the ejection section 30 is slightly shorter than the thickness of the arm section 2. Consequently, the second connection piece string 20 as well as the first connection piece string 21 is sandwiched by the upper roller 312 and the lower roller 311 at a rear part of the ejection section 30. Thereby, the first connection piece string 21 and the second connection piece string 20 are pressed to each other, and joined. The first connection piece string 21 and the second connection piece string 20 which are joined are supported by the plurality of rollers of the ejection section 30, whereby a joined state is kept. When the joined state of the first connection piece string 21 and the second connection piece string 20 is kept, bending of the first connection piece string 21 and the second connection piece string 20 is restricted, and thereby the columnar body having a certain rigidity is formed by the first connection piece string 21 and the second connection piece string 20. Subsequently, the joined arm section 2 is sent out forward from the ejection section 30.

Hereinafter, the ejection section 30 according to a modified example is described with reference to FIGS. 7A and 7B, and 8A to 8C. FIGS. 7A and 7B are cross section views of the ejection section 30 according to the modified example, which are cut along a section orthogonal to the ejection center axis. FIGS. 8A to 8C are diagrams of the ejection section 30 in FIGS. 7A and 7B, which are a top diagram, a side diagram, and a bottom diagram. In the ejection section 30 according to the modified example, ball bearings 342 and 343 are used, in place of the side rollers 313 and 314.

In the ejection section 30 according to the modified example, a plurality of ball bearings 342 and 343 that are arranged in single rows in the respective side wall plates 321 and 322 are fitted to linear receiving grooves 331 and 332 that are formed on side surfaces at both sides of the first connection piece string 21, and thereby the side surfaces of the columnar body are supported.

A pair of receiving grooves 331 and 332 facing each other are formed on outer sides of side surfaces at both sides of the first connection piece 23. The receiving groove 331 has a cross section in an arc shape. The arc has a same diameter as those of balls 318 and 319 that are described later. The pair of receiving grooves 331 and 332 are formed so that a straight line connecting respective deepest portions is perpendicular to the arm center axis. The pair of receiving grooves 331 and 332 form continuous linear receiving grooves when the first connection piece strings 21 are linearly aligned.

The side wall plate 321 includes a plurality of ball bearings 342, and the side wall plate 322 includes a plurality of ball bearings 343. The ball bearing 342 is formed by the ball 318 and a bearing portion 316. The ball 318 is rotatably held by the bearing portion 316. The ball bearing 343 has a same structure as that of the ball bearing 342, and is formed by the ball 319 and a bearing portion 317. The plurality of ball bearings 342 project from the inner side surface of the side wall plate 321, and the plurality of ball bearings 343 project from the inner side surface of the side wall plate 322.

On the side wall plate 321, the plurality of ball bearings 342 are arranged in a row along the ejection center axis. On the side wall plate 322, the plurality of ball bearings 343 are arranged in a row with the columnar body sandwiched between the plurality of ball bearings 343. The plurality of ball bearings 343 are respectively disposed in same positions as those of the plurality of ball bearings 342 with respect to the ejection center axis. The positions at which the plurality of ball bearings 342 and 343 are disposed correspond to positions of the receiving grooves 331 and 332 that are formed on the first connection piece 23. A space between the plurality of ball bearings 342 and the plurality of ball bearings 343 is slightly shorter than a distance between the deepest portions of the receiving grooves 331 and 332. Accordingly, a preload can be applied to between the plurality of ball bearings 342 and 343, and the columnar body. Thereby, a same effect as in the case of using the rollers can be obtained. On the ball bearings 342 and 343 which support the columnar body on the side surfaces, a larger load than that on the upper roller 312 and the lower roller 311 does not work. Accordingly, even when a preload is not applied to between the plurality of ball bearings 342 and 343 and the columnar body, the plurality of ball bearings 342 and 343 can support the columnar body. Accordingly, the space between the plurality of ball bearings 342 and the plurality of ball bearings 343 may be equivalent to the distance between the deepest portions of the receiving grooves 331 and 332.

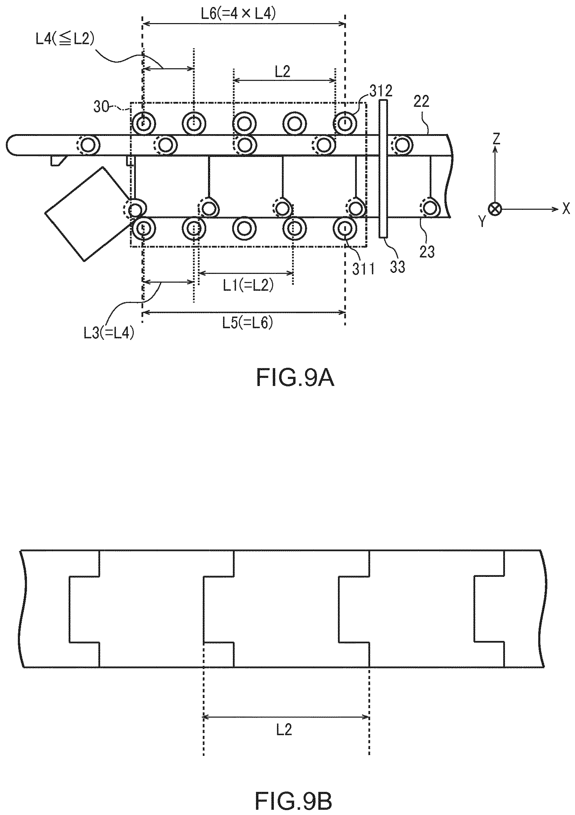

Next, disposition condition of the plurality of upper rollers 312 and the plurality of lower rollers 311 is described with reference to FIGS. 9A and 9B. FIGS. 9A and 9B are supplementary explanatory diagrams for explaining the disposition condition of the plurality of upper rollers 312 and the plurality of lower rollers 311 that form the ejection section 30 according to the present embodiment. FIG. 9A is a diagram of the ejection section 30 of the robot arm mechanism, which is a cross section view. FIG. 9B is a supplementary explanatory diagram for explaining a length of the second connection piece 22. A length of the first connection piece 23 is set as L1 (hereinafter, referred to as a first connection piece length L1), and a length of the second connection piece 22 is set as L2 (hereinafter, referred to as a second connection piece length L2). As shown in FIG. 9B, the length of the second connection piece 22 is a distance from a tip to a rear end of the second connection piece 22 in a direction (a lengthwise direction) in which the second connection pieces 22 are connected. Further, a space between the axes of rotation of the adjacent lower rollers 311 is set as L3 (hereinafter, referred to as a roller space L3), and a space between axes of rotation of the adjacent upper rollers 312 is set as L4 (hereinafter, referred to as a roller space L4). A space between axis of rotation of the leading lower roller 311 of the ejection section 30 and the axis of rotation of the last lower roller 311 is set as L5 (hereinafter, referred to as a lower roller occupied length L5), and a space between the axis of rotation of the leading upper roller 312 of the ejection section 30 and the axis of rotation of the last upper roller 312 is set as L6 (hereinafter, referred to as an upper roller occupied length L6). Here, as a typical example, the first connection piece length L1 corresponds to the second connection piece length L2.

FIGS. 9A and 9B show a typical example of the method of disposing rollers that constitute the ejection section 30. As shown in FIGS. 9A and 9B, the ejection section 30 includes a plurality of lower rollers 311 and a plurality of upper rollers 312, five upper rollers 312 and five lower rollers 311 herein. The plurality of lower rollers 311 and the plurality of upper rollers 312 are respectively disposed in predetermined positions. Specifically, the leading upper roller 312 is disposed in the vicinity of the rear part of the ejection port 33. A direction in which the joined arm section 2 is sent out from the ejection port 33 is set as forward. The other upper rollers 312 are disposed by being separated by the roller space L4 from one another in order from the leading upper roller 312. The roller space L4 is designed to have a length equal to or less than the second connection piece length L2. Thereby, the second connection piece 22 is supported by at least one of the upper rollers 312. For example, the roller space L4 is designed to have a length of 3/5 of the second connection piece length L2.

The second connection piece string 20 is bendable in a front surface direction and a back surface direction thereof. Accordingly, it is possible that the second connection piece string 20 rises in the front surface direction and the back surface direction thereof. However, the front surface of the second connection piece 22 is supported by at least one of the upper rollers 312, so that even when a force that rises in the front surface direction acts on the second connection piece string 20 for some reason, rise in the front surface direction of the second connection piece string 20 can be suppressed. Further, the first connection piece string 21 is joined to the second connection piece string 20 in the back surface direction of the second connection piece string 20. The first connection piece string 21 includes a characteristic that the first connection piece string 21 is unbendable in the front surface direction thereof. Accordingly, even when a force that rises in the back surface direction acts onto the second connection piece string 20 for some reason, rise in the back surface direction of the second connection piece string 20 can be suppressed, because the first connection piece string 21 is unbendable in the front surface direction.

The plurality of lower rollers 311 are respectively disposed in the same positions as those of the plurality of upper rollers 312 with the columnar body sandwiched between the plurality of lower rollers 311 and the plurality of upper rollers 312, with respect to the ejection center axis. That is, the leading upper roller 312 of the plurality of upper rollers 312 is disposed at a same position as that of the leading lower roller 311 of the plurality of lower rollers 311, with respect to the ejection center axis. Further, with respect to the ejection center axis, the last upper roller 312 of the plurality of upper rollers 312 is disposed at a same position as that of the last lower roller 311 of the plurality of lower rollers 311. The upper roller occupied length L6 is equal to the lower roller occupied length L5.

Here, the first connection piece length L1 is equal to the second connection piece length L2. Accordingly, the first connection piece 23 is supported by at least one of the lower rollers 311. The first connection piece string 21 includes a characteristic that the first connection piece string 21 is bendable in the back surface direction thereof, but conversely is unbendable in the front surface direction. Accordingly, the first connection piece string 21 does not rise in the front surface direction. Therefore, the roller space L3 does not have to have the length equal to or less than the first connection piece length L1. Further, the second connection piece string 20 is joined to the first connection piece string 21 in the back surface direction of the first connection piece string 21. Rise in the front surface direction of the second connection piece string 20 is suppressed by the plurality of upper rollers 312. Accordingly, even when a force that rises in the back surface direction of the first connection piece string 21 acts on the first connection piece string 21 for some reason, the first connection piece string 21 does not rise in the back surface direction, because the second connection piece string 20 does not rise in the front surface direction.

Summing up the above, by disposing the plurality of upper rollers 312 so that the roller space L4 has the length equal to or less than the second connection piece length L2, the second connection piece 22 is supported by at least one of the upper rollers 312. Thereby, it is possible to suppress rise in the front surface direction of the second connection piece string 20. Further, as a result that the rise in the front surface direction of the second connection piece string 20 is suppressed, it becomes possible to suppress rise in the front surface direction of the first connection piece string 21. Furthermore, the first connection piece string 21 includes the characteristic that the first connection piece string 21 is unbendable in the front surface direction, whereby it becomes possible to suppress rise in the front surface directions of the first connection piece string 21 and the second connection piece string 20. It is preferable that a similar condition to the condition of the plurality of upper rollers 312 is applied to the plurality of side rollers 313 and 314. That is, it is preferable that the plurality of side rollers 313 and 314 are disposed so that each of distances between axes of rotation of the adjacent side rollers becomes the length equal to or less than the second connection piece length L2. Thereby, a deviation in the turning direction of the arm section 2 can be prevented. As a result, it is possible to enhance rigidity of the ejection section 30.

Further, the plurality of lower rollers 311 are respectively disposed in the same positions as the positions of the plurality of upper rollers 312 with the columnar body sandwiched between the plurality of lower rollers 311 and the plurality of upper rollers 312, with respect to the ejection center axis, and thereby the effect as follows is provided. That is, in the upper roller 312 and the lower roller 311 that face each other with the columnar body sandwiched between the upper roller 312 and the lower roller 311, a direction of a load that is given to the columnar body by the upper roller 312 is linear with a direction of a load that is given to the columnar body by the lower roller 311. Consequently, a moment does not work onto the columnar body by the plurality of upper rollers 312 and the plurality of lower rollers 311. Accordingly, elastic deformation of the columnar body is suppressed, and extension and retraction motion of the arm section 2 can be made smooth, as compared with a case where the plurality of lower rollers 311 are disposed at different positions from the positions of the plurality of upper rollers 312 with the columnar body sandwiched between the plurality of lower rollers 311 and the plurality of upper rollers 312, with respect to the ejection center axis. As a result, it is possible to further enhance the rigidity of the ejection section 30, as compared with the case where the plurality of lower rollers 311 are disposed in different positions from the positions of the plurality of upper rollers 312 with the columnar body sandwiched between the plurality of lower rollers 311 and the plurality of upper rollers 312, with respect to the ejection center axis. It does not deny that the plurality of lower rollers 311 are disposed in the different positions from the positions of the plurality of upper rollers 312 with the columnar body sandwiched between the plurality of lower rollers 311 and the plurality of upper rollers 312, with respect to the ejection center axis.

A lower limit of the roller space L4 is determined based on a diameter of the roller. That is, the roller space L4 is longer than a diameter of the upper roller 312. By reducing the roller space L4, it can be made possible to reduce a possibility of the second connection piece string 20 rising.

Further, when the roller space L4 is reduced while the number of upper rollers 312 is kept, for example, the upper roller occupied length L6 becomes short. Then, a range in which the ejection section 30 can support the columnar body is narrowed, the force that holds the arm section 2 by the ejection section 30 becomes small, and the rigidity of the ejection section 30 may be reduced. In the case like this, it is necessary to increase a preload that works between the upper rollers 312 and the lower rollers 311 of the ejection section 30, and the columnar body. However, a magnitude of the preload that is applied between the single roller and the columnar body has an upper limit value. Accordingly, even if the preload is applied to the maximum limit, the rigidity of the ejection section 30 may be low. That is, in order to keep the force that holds the arm section 2, of the ejection section 30, while the roller space L4 is made short, it is necessary to increase the number of upper rollers 312. Then, the number of components increases, and cost increases. That is, the number of rollers constituting the ejection section 30 and the roller space are designed so as to be able to keep the force that holds the arm section 2 at a fixed force or more, prevent rise of the first connection piece string 21 and the second connection piece string 20, and join the first connection piece string 21 and the second connection piece string 20.

Further, the last lower roller 311 does not have to be disposed at a same position as the position of the last upper roller 312 with respect to the ejection center axis. In the arm section 2 according to the present embodiment, joining of the first connection piece string 21 and the second connection piece string 20 is realized by sandwiching a lock portion included by the second connection piece 22, between the adjacent first connection pieces 23.

It is assumed that the last lower roller 311 is disposed forward of the last upper roller 312 with respect to the ejection center axis. At this time, the second connection piece string 20 is linearly aligned earlier than the first connection piece string 21. When the first connection piece string 21 is pressed to the second connection piece string 20 that is linearly aligned, the adjacent first connection pieces 23 can sandwich the lock portion included by the second connection piece 22. Consequently, the last lower roller 311 may be disposed forward of the last upper roller 312.

It is assumed that the last lower roller 311 is disposed rearward of the last upper roller 312 with respect to the ejection center axis. At this time, the first connection piece string 21 is linearly aligned earlier than the second connection piece string 20. When the second connection piece string 20 is pressed to the first connection piece string 21 that is linearly aligned, the adjacent first connection pieces 23 cannot sandwich the lock portion included by the second connection piece 22. Consequently, the last lower roller 311 should not be disposed rearward of the last upper roller 312. However, this does not deny all cases in which the last lower roller 311 is disposed rearward of the last upper roller 312. The last lower roller 311 may be disposed rearward of the last upper roller 312, as long as the adjacent first connection pieces 23 passing through the last lower roller 311 can sandwich the lock portion included by the second connection piece 22.

The structure of the ejection section 30 is not limited to the structure in FIGS. 9A and 9B, as long as the ejection section 30 can keep the support function and the joining function.

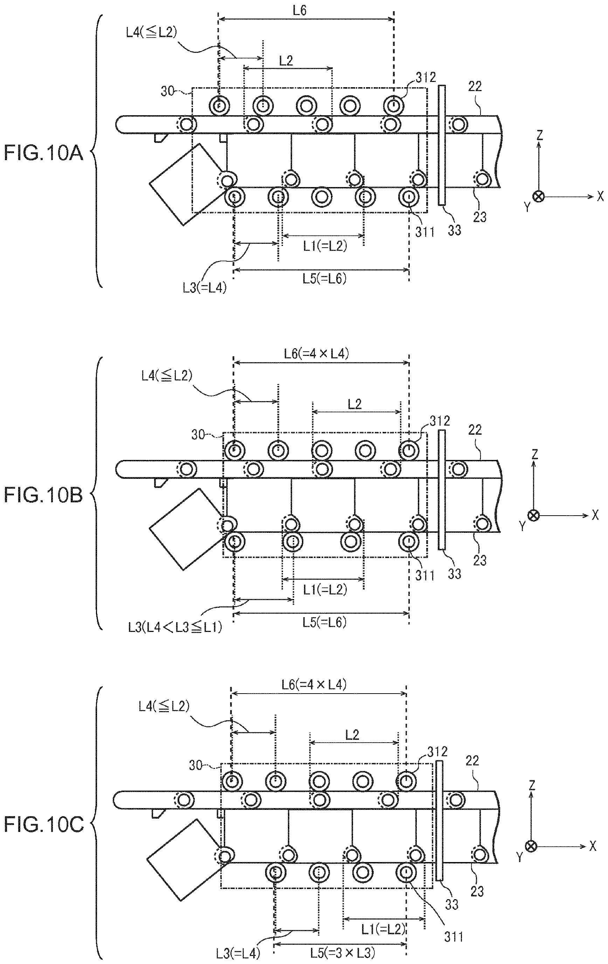

FIGS. 10A to 10C are views illustrating modified examples of the ejection section 30 in FIGS. 9A and 9B.

As shown in FIG. 10A, for example, the plurality of lower rollers 311 may be respectively disposed in different positions with respect to the ejection center axis from the positions of the plurality of upper rollers 312 with the columnar body sandwiched between the plurality of lower rollers 311 and the plurality of upper rollers 312. At this time, the last lower roller 311 is disposed at the same position as the position of the last upper roller 312 or forward of the last upper roller 312 with respect to the ejection center axis. Even when the plurality of lower rollers 311 and the plurality of upper rollers 312 are disposed as in FIG. 10A, it is sufficiently possible to join the first connection piece string 21 and the second connection piece string 20 and support the columnar body. That is, it is possible to enhance the rigidity of the ejection section 30.

Further, as shown in FIGS. 10B and 10C, the number of lower rollers 311 may be smaller than the number of upper rollers 312. Thereby, it is possible to reduce the number of components forming the ejection section 30, as compared with the structure in FIGS. 9A and 9B. At this time, as shown in FIG. 10B, in order to make the lower roller occupied length L5 equal to the upper roller occupied length L6, the plurality of lower rollers 311 are arranged so that the roller space L3 is longer than the roller space L4. In other words, the plurality of lower rollers 311 are disposed so that the leading lower roller 311 faces the leading upper roller 312 with the columnar body sandwiched between the leading lower roller 311 and the leading upper roller 312, and the last lower roller 311 faces the last upper roller 312 with the columnar body between the last lower roller 311 and the last upper roller 312. Thereby, the range in which the ejection section 30 can support the columnar body can be made substantially equivalent to the range in the structure of the ejection section 30 in FIGS. 9A and 9B, and as a result, it becomes possible to support the columnar body with a substantially equivalent force to the force in the structure in FIGS. 9A and 9B, and hold the arm section 2. Further, as shown in FIG. 10C, the plurality of lower rollers 311 may be arranged in order from the leading lower roller 311 so that the roller space L3 has an equivalent length to the length of the roller space L4. Thereby, with respect to the ejection center axis, the plurality of lower rollers 311 are respectively disposed at the same positions as the positions of the plurality of upper rollers 312 with the columnar body sandwiched between the plurality of lower rollers 311 and the plurality of upper rollers 312, so that elastic deformation of the columnar body can be suppressed, and the extension and retraction motion of the arm section 2 can be made smooth, as compared with the structure in FIG. 10B. Though not illustrated, the number of upper rollers 312 may be smaller than the number of lower rollers 311.

As the typical example of the ejection section 30, the case in which the ejection section 30 includes the plurality of upper rollers 312 and the plurality of lower rollers 311 is described as the example thus far. However, the ejection section 30 may be formed of the single upper roller 312 and the single lower roller 311, as long as the ejection section 30 supports the columnar body, and can join the first connection piece string 21 and the second connection piece string 20.

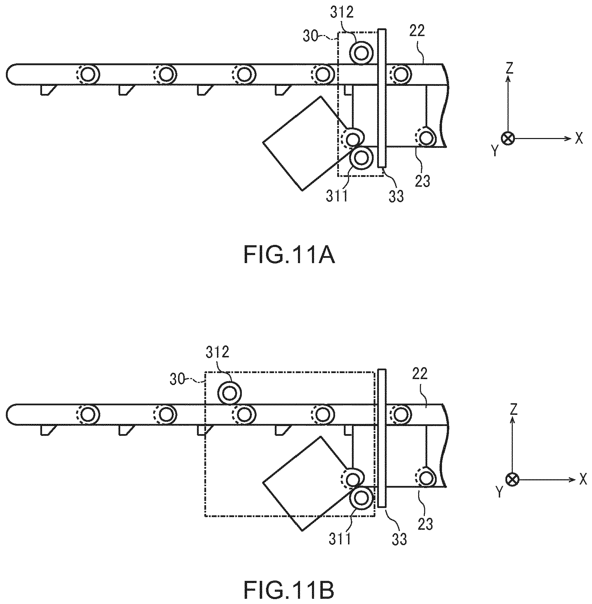

FIGS. 11A and 11B are views illustrating examples of a structure of the ejection section 30 in the case of using the single upper roller 312 and the single lower roller 311. As shown in FIGS. 11A and 11B, the ejection section 30 includes the single upper roller 312 and the single lower roller 311. The single lower roller 311 is disposed in the vicinity of the ejection port 33. As shown in FIG. 11A, the single upper roller 312 is disposed in the same position as the position of the single lower roller 311 with the columnar body sandwiched between the single upper roller 312 and the single lower roller 311, with respect to the ejection center axis. A preload is applied to between the lower roller 311 and the upper roller 312, and the columnar body. Thereby, even the single lower roller 311 and the single upper roller 312 can enhance the rigidity of the ejection section 30. That is, the deformation amount of the ejection section 30 can be reduced with respect to an exerted external force accompanying movement of the arm section 2 and the end effector 3, and an external force that works in accordance with the weights of the arm section 2 and the end effector 3. Thereby, it is possible to enhance precision with which the ejection section 30 keeps the position of the arm section 2.

Further, when a gravity direction is a -Z direction in the drawings, as shown in FIG. 11B, the single upper roller 312 may be disposed rearward of the single lower roller 311 with respect to the ejection center axis. A moment of a force occurs to the columnar body due to the gravity that works in accordance with the weights of the arm section 2 and the end effector 3. By disposing the single upper roller 312 rearward of the single lower roller 311, the columnar body that is to rotate by the moment can be suppressed. Thereby, the ejection section 30 can hold the position of the arm section 2 as in FIG. 11A.

According to the ejection section 30 of the robot arm mechanism according to the present embodiment, which is described above, the following effects are obtained. The ejection section 30 includes the upper roller 312 and the lower roller 311. The upper roller 312 and the lower roller 311 are disposed with the columnar body sandwiched between the upper roller 312 and the lower roller 311. At this time, the preload is applied to between the upper roller 312 and the lower roller 311, and the columnar body. In order to apply the preload between the upper roller 312 and the lower roller 311, and the columnar body, the distance between the upper roller 312 and the lower roller 311 concerning the thickness direction is shorter than the thickness of the columnar body, for example. The preload is applied to between the columnar body and the ejection section 30, whereby it is possible to suppress rattling between the ejection section 30 and the columnar body, and enhance the rigidity of the ejection section 30. Thereby, it is possible to enhance precision for holding the arm section 2 with the ejection section 30.

Further, the first connection piece string 21 and the second connection piece string 20 that are guided to the ejection section 30 are sandwiched by the last upper roller 312 and the last lower roller 311, are pressed to each other, and are joined to each other. By the joining function of the ejection section 30, the columnar body can be formed. When the ejection section 30 is formed of the single upper roller 312 and the single lower roller 311, the similar effect can be obtained. Accordingly, the ejection section 30 of the robot arm mechanism according to the present embodiment includes the function of joining the first connection piece string 21 and the second connection piece string 20 that are guided to the ejection section 30 and the function of supporting the arm section 2.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

### Reference Signs List

1..Base, 2...Arm section, 3...End effector, J1, J2, J4, J5, J6...Rotary joint, J3...Linear motion joint, 11a...First support body, 11b...Second support body, 11c...Third support body, 20...Second connection piece string, 21...First connection piece string, 22...Second connection piece, 23...First connection piece, 26...Joining piece, 30...Ejection section, 311...Lower roller, 312...Upper roller, 313, 314...Side roller, 321, 322...Side wall plate, 323...Lower wall plate, 324...Upper wall plate, 325, 326, 327, 328...Bearing portion

## Claims

1. A robot arm mechanism comprising a linear extension and retraction joint, wherein
the linear extension and retraction joint includes an arm section, and an ejection section for supporting the arm section,
the arm section includes a first connection piece string and a second connection piece string,
the first connection piece string includes a plurality of first connection pieces each having a U-shaped cross section or a hollow square cross section, the second connection piece string includes a plurality of second connection pieces each having a substantially flat plate shape, and the second connection piece string is joined to the first connection piece string to thereby constitute a columnar body having a certain rigidity, and
the ejection section includes first rollers and second rollers for joining the first and second connection piece strings and supporting the columnar body by joining of the first and second connection piece strings, and the first rollers and the second rollers are disposed with the columnar body sandwiched between the first rollers and the second rollers.

2. The robot arm mechanism according to claim 1, wherein
the first rollers support the columnar body at a side of the first connection pieces, and the second rollers support the columnar body at a side of the second connection pieces.

3. The robot arm mechanism according to claim 1, wherein
the first and second rollers are provided so that a preload is applied to between the first and second rollers, and the columnar body.

4. The robot arm mechanism according to claim 3, wherein
a distance between the first rollers and the second rollers with respect to a thickness direction of the arm section is shorter than a thickness of the arm section.

5. The robot arm mechanism according to claim 2, wherein
the second rollers are arranged so that a distance between axes of rotation has a length equal to or less than a length of the second connection piece.

6. The robot arm mechanism according to claim 2, wherein
the second rollers are provided at same positions as positions of the first rollers, or rearward of the first roller, with respect to a center axis of the arm section.

7. The robot arm mechanism according to claim 5, wherein
a same number of the first rollers as the second rollers are provided.

8. The robot arm mechanism according to claim 7, wherein
the first rollers are disposed at same positions as the second rollers with respect to a center axis of the arm section.

9. The robot arm mechanism according to claim 5, wherein
the first rollers are provided at different positions from positions of the second rollers with respect to a center axis of the arm section.

10. The robot arm mechanism according to claim 5, wherein
a smaller number of the first rollers than the second rollers are provided.

11. The robot arm mechanism according to claim 1, wherein
the ejection section includes third rollers and fourth rollers that are disposed with the columnar body sandwiched between the third rollers and the fourth rollers, and the third rollers are disposed at different positions from positions of the fourth rollers with respect to a width direction of the columnar body.

12. The robot arm mechanism according to claim 1, wherein
the ejection section includes ball bearings disposed with the arm section sandwiched between the ball bearings to support the columnar body, and
receiving grooves for receiving the ball bearings are formed on both side surfaces of the first connection piece.
